(19) Europäisches Patentamt
European Patent Office
Office européen des brevets

(11) Veröffentlichungsnummer : **0 136 647**
**B1**

(12) **EUROPÄISCHE PATENTSCHRIFT**

(45) Veröffentlichungstag der Patentschrift : **20.01.88**

(51) Int. Cl.[4] : **C 07 D 335/02**, A 01 N 43/18

(21) Anmeldenummer : **84111390.5**

(22) Anmeldetag : **25.09.84**

(54) **Cyclohexenonderivate, Verfahren zu ihrer Herstellung sowie ihre Verwendung zur Bekämpfung unerwünschten Pflanzenwuchses.**

(30) Priorität : **30.09.83 DE 3335455**
**05.04.84 DE 3412794**

(43) Veröffentlichungstag der Anmeldung : **10.04.85 Patentblatt 85/15**

(45) Bekanntmachung des Hinweises auf die Patenterteilung : **20.01.88 Patentblatt 88/03**

(84) Benannte Vertragsstaaten : **AT BE CH DE FR GB IT LI NL SE**

(56) Entgegenhaltungen :
**EP-A- 0 071 707**
**EP-A- 0 115 808**

(73) Patentinhaber : **BASF Aktiengesellschaft**
**Carl-Bosch-Strasse 38**
**D-6700 Ludwigshafen (DE)**

(72) Erfinder : **Becker, Rainer, Dr.**
**Im Haseneck 22**
**D-6702 Bad Duerkheim (DE)**
Erfinder : **Jahn, Dieter, Dr.**
**Burgunder Weg 8**
**D-6803 Edingen-Neckarhausen (DE)**
Erfinder : **Keil, Michael, Dr.**
**Fontanestrasse 4**
**D-6713 Freinsheim (DE)**
Erfinder : **Schirmer, Ulrich, Dr.**
**Berghalde 79**
**D-6900 Heidelberg (DE)**
Erfinder : **Wuerzer, Bruno, Dr. Dipl.-Landwirt**
**Ruedigerstrasse 13**
**D-6701 Otterstadt (DE)**
Erfinder : **Meyer, Norbert, Dr.**
**Dossenheimer Weg 22**
**D-6802 Ladenburg (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 136 647 B1

Jouve, 18, rue St-Denis, 75001 Paris, France

**Beschreibung**

Die vorliegende Erfindung betrifft Cyclohexenonderivate, Verfahren zu ihrer Herstellung sowie Herbizide, die diese Verbindungen als Wirkstoffe enthalten.

Es ist bekannt, daß Cyclohexan-1,3-dionderivate mit heterocyclischen Substituenten in 5-Stellung und Halogenalkenyloxyamino-alkylidenresten in 2-Stellung herbizid wirksam sind (EP-OS 00 71 707). 5-Tetrahydrothiopyranyl-cyclohexandionderivate mit Halogenalkenylresten als Oximethersubstituenten sind jedoch nicht beschrieben.

Es wurde gefunden, daß Cyclohexenonderivate der Formel

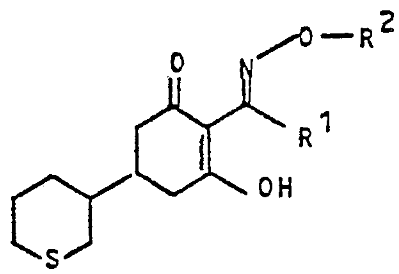

(I)

in der

$R^1$ $C_1$-$C_4$-Alkyl und

$R^2$ $C_3$-$C_5$-Chloralkenyl bedeuten,

und ihre Salze herbizid ausgezeichnet wirksam sind.

Die Verbindungen der Formel I können in tautomeren Formen auftreten, die alle vom Patentanspruch umfaßt werden :

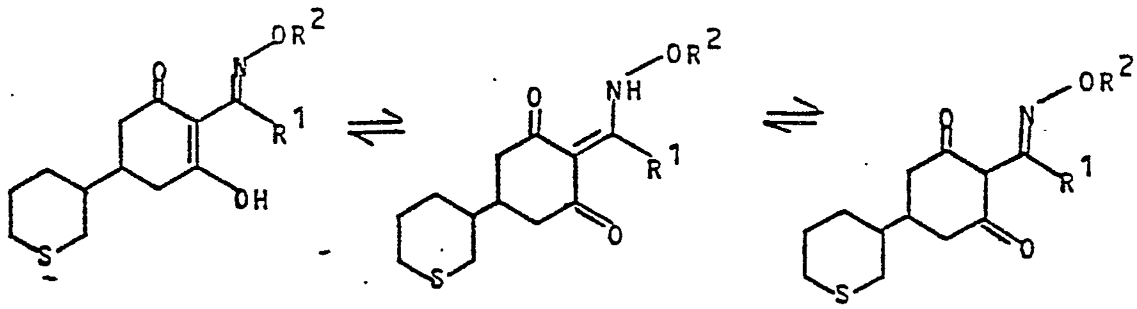

In der allgemeinen Formel I bedeuten

$R^1$ Methyl, Ethyl, n-Propyl, i-Propyl, n-Butyl, s-Butyl, t-Butyl oder i-Butyl und

$R^2$ einen 1 bis 3 Chloratome tragenden $C_3$-$C_5$-Alkenylrest, beispielsweise cis-3-Chlorallyl, trans-3-Chlorallyl, cis-3-Chlorbut-2-enyl, trans-3-Chlorbut-2-enyl, 2-Chlorallyl oder 2,3,3-Trichlor-prop-2-enyl.

Bevorzugte Cyclohexenonderivate der Formel I sind solche, bei denen $R^2$ einen 3-Chlor-prop-2-enylrest bedeutet. Bevorzugte Reste für $R^1$ sind Methyl, Ethyl, n-Propyl, vorzugsweise Ethyl und n-Propyl.

Als Salze der Verbindungen der Formel I kommen beispielsweise die Alkalimetallsalze, insbesondere die Kalium- oder Natriumsalze, Erdalkalimetallsalze, insbesondere Calcium-, Magnesium- oder Bariumsalze sowie Mangan-, Kupfer-, Zink- oder Eisensalze und Ammonium- und Phosphoniumsalze, beispielsweise Alkylammonium-, Dialkylammonium-, Trialkyl- oder Tetraalkylammoniumsalze, Benzyltrialkylammoniumsalze, Triphenylphosphoniumsalze, Trialkylsulfoniumsalze oder Trialkylsulfoxoniumsalze in Betracht.

Die Verbindungen der Formel I können durch Umsetzung von Verbindungen der Formel

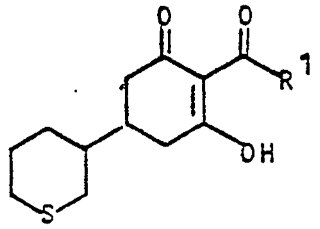

(III)

in der $R^1$ die obengenannte Bedeutung hat, mit Hydroxylaminderivaten $R^2O$—$NH_3Y$, in der $R^2$ die obengenannten Bedeutungen hat und Y ein Anion bedeutet, erhalten werden.

Man führt die Reaktion zweckmäßigerweise in heterogener Phase in einem inerten Verdünnungsmittel bei einer Temperatur zwischen 0 und 80 °C oder zwischen 0 °C und dem Siedepunkt des Reaktionsgemisches in Gegenwart einer Base durch. Geeignete Basen sind beispielsweise Carbonate, Hydrogencarbonate, Acetate, Alkoholate, Hydroxide oder Oxide von Alkali- oder Erdalkalimetallen, insbesondere von Natrium, Kalium, Magnesium, Calcium.

Außerdem können auch organische Basen, wie Pyridin oder tertiäre Amine, Verwendung finden.

Die Umsetzung verläuft besonders gut in einem pH-Bereich von 2 bis 9, insbesondere von 4,5 bis 5,5. Die Einstellung des pH-Bereiches erfolgt zweckmäßigerweise durch Zusatz von Acetaten, beispielsweise Alkalimetallacetaten, insbesondere von Natrium- oder Kaliumacetat oder einer Mischung aus beiden Salzen. Alkalimetallacetate werden beispielsweise in Mengen von 0,5 bis 2 Mol, bezogen auf die Ammoniumverbindung der Formel $R^2O—NH_3Y$, zugesetzt.

Als Lösungsmittel sind beispielsweise Dimethylsulfoxid, Alkohole, wie Methanol, Ethanol, Isopropanol, Benzol, gegebenenfalls chlorierte Kohlenwasserstoffe, wie Chloroform, Dichlorethan, Hexan, Cyclohexan, Ester, wie Essigsäureethylester, Ether, wie Dioxan, Tetrahydrofuran, geeignet.

Die Umsetzung ist nach wenigen Stunden beendet, das Reaktionsprodukt kann dann durch Einengen der Mischung, Zugabe von Wasser und Extraktion mit einem unpolaren Lösungsmittel, wie Methylenchlorid, und Abdestillieren des Lösungsmittels unter vermindertem Druck isoliert werden.

Die Verbindungen der Formel I können außerdem durch Umsetzen der Verbindungen der Formel II mit Hydroxylaminen der Formel $R^2O—NH_2$, in der $R^2$ die obengenannten Bedeutungen hat, in inerten Verdünnungsmitteln bei einer Temperatur zwischen 0 °C und dem Siedepunkt des Reaktionsgemisches, insbesondere zwischen 15 und 70 °C, erhalten werden. Gegebenenfalls kann das Hydroxylamin in Form einer wäßrigen Lösung eingesetzt werden.

Geeignete Lösungsmittel für diese Umsetzung sind beispielsweise Alkohole, wie Methanol, Ethanol, Isopropanol, Cyclohexanol, gegebenenfalls chlorierte Kohlenwasserstoffe, wie Hexan, Cyclohexan, Methylenchlorid, Toluol, Dichlorethan, Ester, wie Essigsäureethylester, Nitrile, wie Acetonitril, cyclische Ether, wie Tetrahydrofuran.

Die Verbindungen der Formel I können weiterhin durch Umsetzen von Verbindungen der Formel II mit unsubstituiertem Hydroxylammoniumsalz $NH_2OH \cdot HY$, wobei Y die obige Bedeutung hat, bei 0 bis 80 °C in Gegenwart eines Lösungsmittels und einer Hilfsbase zu einem entsprechenden Oxim umgesetzt und dann mit einem Alkylierungsmittel $R^2$-Y', wobei Y' eine Abgangsgruppe bedeutet und $R^2$ die obige Bedeutung hat, O-alkyliert werden. Dabei muß man die Neigung der als Zwischenprodukte gebildeten Oxime zu unerwünschten Cyclisierungsreaktionen beachten ; durch geeignete Hilfsstoffe und Reaktionsbedingungen läßt sich dies beeinflussen.

Geeignete Lösungsmittel sind die für die Umsetzung der Verbindungen der Formel II mit Hydroxylaminen aufgeführten Lösungsmittel, geeignete Hilfsbasen sind die für die Umsetzung der Verbindungen der Formel II mit Hydroxylaminderivaten der Formel $R^2—O—NH_3Y$ genannten basischen Substanzen, wobei allerdings die doppelte Menge an Base erforderlich ist.

Die Alkalimetallsalze der Verbindungen der Formel I können durch Behandeln dieser Verbindungen mit Natrium- oder Kaliumhydroxid in wäßriger Lösung oder in einem organischen Lösungsmittel, wie Methanol, Ethanol, Aceton, erhalten werden. Auch Natrium- und Kaliumalkoholate können als Basen dienen.

Die anderen Metallsalze, z. B. die Mangan-, Kupfer-, Zink-, Eisen-, Calcium-, Magnesium- und Bariumsalze, können aus den Natriumsalzen durch Reaktion mit den entsprechenden Metallchloriden in wäßriger Lösung hergestellt werden. Ammonium- und Phosphoniumsalze können durch Umsetzung von Verbindungen der Formel I mit Ammonium-, Phosphonium-, Sulfonium- oder Sulfoxoniumhydroxiden gegebenenfalls in wäßriger Lösung hergestellt werden.

Die Verbindungen der Formel II können aus Cyclohexan-1,3-dionen der Formel IV, die auch in der tautomeren Form IV a vorliegen können,

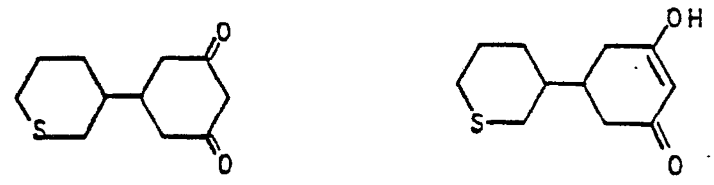

(IV) (IVa)

nach literaturbekannten Methoden (Tetrahedron Letters, 29, 2491 (1975)) hergestellt werden.

Es ist auch möglich, Verbindungen der Formel II über die Zwischenstufe der Enolester, die bei der Umsetzung von Verbindungen der Formel IV anfallen und in Gegenwart von Imidazol- oder Pyridinderivaten umgelagert werden (JP-OS 79/063052), herzustellen.

Zu den Verbindungen der Formel IV gelangt man nach literaturbekannten Verfahren, wie dies aus folgendem Schema hervorgeht :

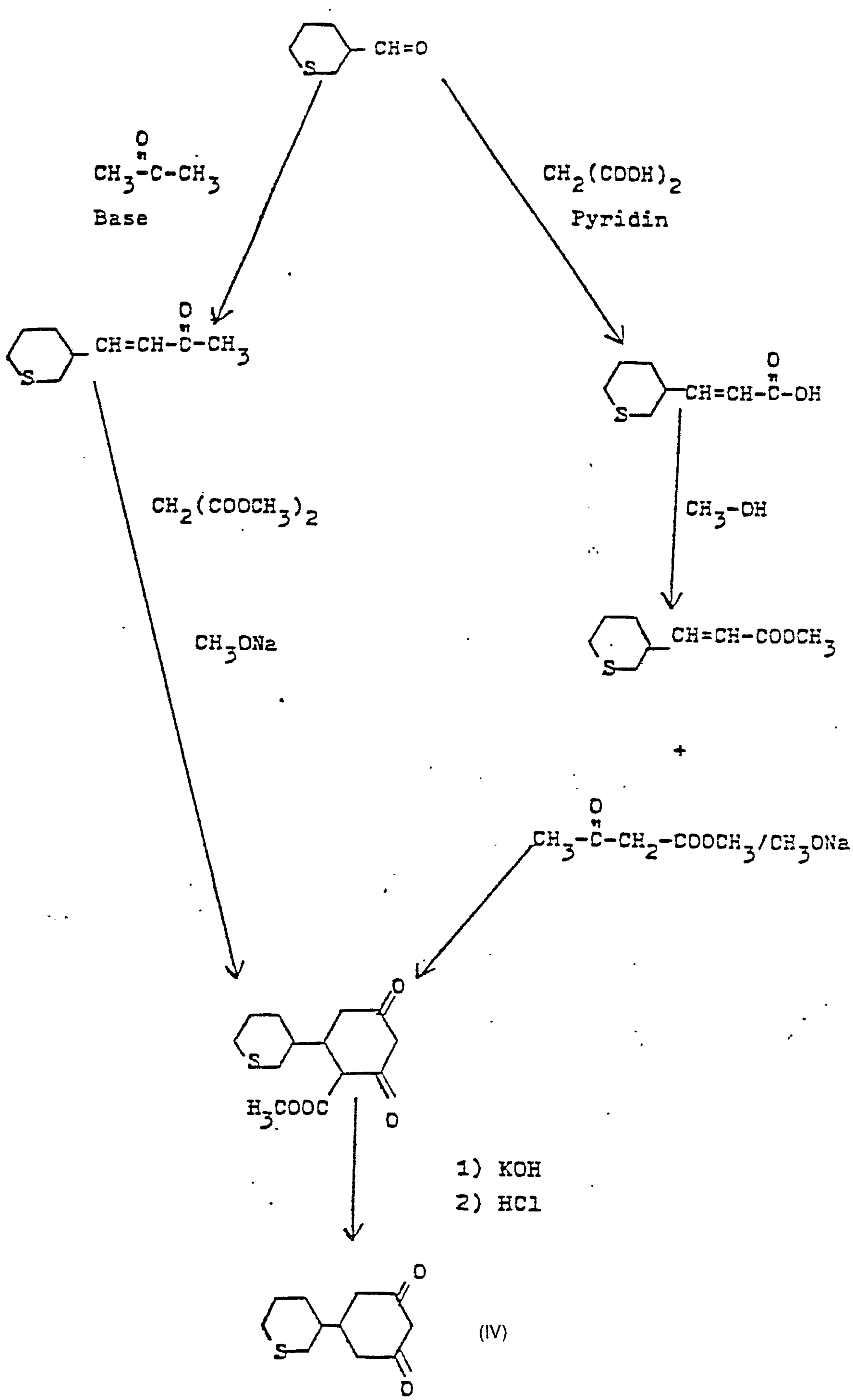


(IV)

Das folgende Beispiel erläutert die Herstellung der Cyclohexenonderivate der Formel I. Dabei verhalten sich Gewichtsteile zu Volumenteilen wie Kilogramm zu Liter.

Beispiel 1

14,1 Gewichtsteile 2-(n-Butyryl)-5-(tetrahydrothiopyran-3-yl)-cyclohexan-1,3-dion werden in 100 Volumenteilen Methanol gelöst und mit 7,9 Gewichtsteilen 0-(trans-3-Chlorallyl)-hydroxylaminhydrochlorid sowie 4,6 Gewichtsteilen Natriumhydrogencarbonat versetzt und einige Stunden bei Raumtemperatur gerührt; anschließend wird eingeengt, in Methylchlorid aufgenommen, mit Wasser gewaschen und wieder eingeengt. Es verbleiben 16,1 Gewichtsteile eines Öls vom $n_D^{22} = 1{,}5669$ (Verbindung Nr. 1).

Die folgenden Verbindungen der Formel I lassen sich auf analogem Wege herstellen:

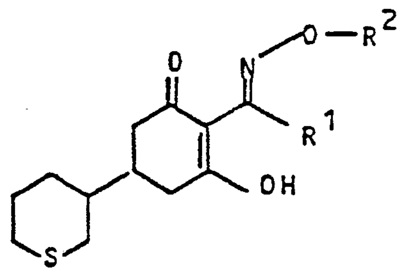

| Verbindung Nr. | $R^1$ | $R^2$ | $n_D$/Fp [°C]/$^1$HNMR-Daten (in ppm, bezogen auf Tetramethylsilan) |
|---|---|---|---|
| 1 | $C_3H_7$ | trans-$CH_2CH{=}CHCl$ | 1,5669 (22 °C) |
| 2 | $C_3H_7$ | cis-$CH_2CH{=}CHCl$ | 1,5661 (22 °C) |
| 3 | $C_2H_5$ | trans-$CH_2CH{=}CHCl$ | 1,5730 (22 °C) |
| 4 | $C_2H_5$ | cis-$CH_2CH{=}CHCl$ | 1,5715 (22 °C) |
| 5 | $CH_3$ | trans-$CH_2CH{=}CHCl$ | 57-64 |
| 6 | $CH_3$ | cis-$CH_2CH{=}CHCl$ | 48-50 |
| 7 | n-$C_3H_7$ | $CH_2CCl{=}CH_2$ | |
| 8 | $C_2H_5$ | $CH_2CCl{=}CH_2$ | |
| 9 | n-$C_3H_7$ | trans-$CH_2CH{=}CClCH_3$ | |
| 10 | n-$C_3H_7$ | cis-$CH_2CH{=}CClCH_3$ | |
| 11 | $C_2H_5$ | trans-$CH_2CH{=}CClCH_3$ | |
| 12 | n-$C_3H_7$ | cis-$CH_2CH{=}CClCH_3$ | |
| 13 | n-$C_3H_7$ | $CH_2CCl{=}CCl_2$ | 1,5704 (24°C) |

Die Cyclohexenonderivate der Formel I und ihre Salze können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden.

Die Anwendungsformen richten sich ganz nach den Verwendungszwecken ; sie sollten in jedem Fall möglichst die feinste Verteilung der Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind :

I. Man vermischt 90 Gewichtsteile der Verbindung Nr. 13 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 4 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile des Wirkstoffs Nr. 1 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile des Wirkstoffs Nr. 3 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile des Wirkstoffs Nr. 3 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile des Wirkstoffs Nr. 6 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile des Wirkstoffs Nr. 5 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,01 bis 3 kg/ha, vorzugsweise 0,025 bis 0,5 kg/ha.

Die Wirkung der Cyclohexenonderivate der Formel I auf das Wachstum von Pflanzen aus der Gräserfamilie (Gramineen) und breitblättrigen Kulturpflanzen läßt sich durch Gewächshausversuche zeigen:

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät.

Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmengen betragen 3,0 kg/ha.

Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung werden die Testpflanzen je nach Wuchsform erst bis zu einer Wuchshöhe von 3 bis 15 cm angezogen und dann mit den Wirkstoffen bzw. den herbiziden Mitteln behandelt. Die Sojapflanzen werden in einem mit Torfmull (peat) angereicherten Substrat angezogen. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmengen für die Nachauflaufbehandlung variieren zwischen 0,015 und 3 kg Wirkstoff/ha. Eine Abdeckung unterbleibt bei der Nachauflaufbehandlung.

Sowohl für die Vorauflauf- als auch die Nachauflaufbehandlung werden die Versuchsgefäße im Gewächshaus aufgestellt, wobei für wärmeliebende Arten wärmere Bereiche (20 bis 35 °C) und für solche gemäßigter Klimate 10 bis 25 °C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächschausversuchen verwendeten Pflanzen setzen sich aus folgenden Arten zusammen:

Avena sativa (Hafer), Echinochloa crus-galli (Hühnerhirse), Glycine max (Sojabohnen), Hordeum vulgare (Gerste), Lolium multiflorum (Ital. Ray-gras), Setaria italica (Kolbenhirse), Sinapis alba (Weißer Senf), Sorghum bicolor (Mohrenhirse), Sorghum halepense (Sudangras), Triticum aestivum (Weizen), Zea mays (Mais), Centaurea cyanus (Kornblume), Cyperus esculentus (Erdmantel), Galium aparine (Klettenlabkraut), Ipomoea spp. (Prunkwindearten), Mercurialis annua (Einjähriges Bingelkraut).

Als Vergleichsmittel werden die aus der EP-OS 00 71 707 bekannten Verbindungen der Formel

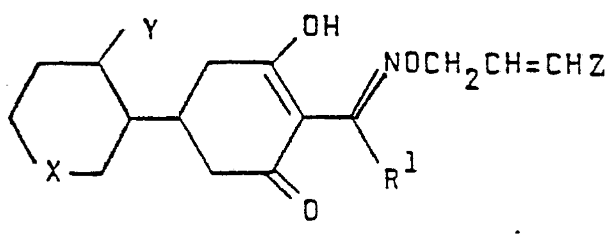

(Fortsetzung Seite 8 f.)

| Verbindung Nr. | $R^1$ | X | Y | Z |
|---|---|---|---|---|
| I | $C_2H_5$ | S | H | H |
| II | $n-C_3H_7$ | O | $CH_3$ | Cl |
| III | $n-C_3H_7$ | O | H | Cl |

herangezogen.

Vorauflaufanwendung

Bei Vorauflaufanwendung sind beispielsweise die Verbindungen Nr. 1, 2 und 4 bei einer Aufwandmenge von 3,0 kg Wirkstoff/ha herbizid wirksam gegen Pflanzen aus der Familie der Gräser (Gramineen) ; dabei bleibt Sinapis alba als breitblättrige Testpflanze unbeschadet.

Nachauflaufanwendung

Bei Nachauflaufanwendung zeigt die Verbindung Nr. 1 mit 0,06 kg Wirkstoff/ha eine dem Vergleichsmittel II und mit 0,125 kg Wirkstoff/ha eine dem Vergleichsmittel III deutlich überlegene herbizide Wirkung gegen Pflanzen aus der Familie der Gramineen. An Sojapflanzen tritt hierbei keine Schädigung auf. Die Verbindung Nr. 3 wirkt mit 0,015 kg Wirkstoff/ha stärker als das Vergleichsmittel I gegen Gräser, wie Echinochloa crus-galli oder Sorghum bicolor (als Ausfallhirse oder « shattercane »). Bei Nachauflaufanwendung von 0,125 kg Wirkstoff/ha zeigt diese Verbindung eine viel stärkere Aktivität gegen Weizen (der auch als unerwünschtes Gras auftreten kann) als das Vergleichsmittel I, bei gleichzeitiger Verträglichkeit für Soja.

Im Gegensatz dazu schädigen die Verbindungen Nr. 2 und 4 mit 0,06 kg Wirkstoff/ha den Weizen (Triticum aestivum, Sorte « Vuka ») nur geringfügig und sind somit für diese Kultur als selektiv zu betrachten, während das Vergleichsmittel III erhebliche Schäden verursacht. Die Verträglichkeit für breitblättrige Kulturen ist ebenfalls vorhanden.

Bei Aufwandmengen von 3,0 kg/ha haben die Verbindungen Nr. 5, 6 und 13 eine ausgezeichnete Aktivität gegen Arten aus der Familie der Gräser, die Verbindungen Nr. 5 und 6 haben dabei außerdem eine beachtliche Wirkung gegen Cyperus esculentus und beispielhaft ausgewählte breitblättrige Unkräuter.

In Anbetracht der Verträglichkeit der Applikationsmethoden können die Cyclohexenonderivate der Formel I noch in einer weiteren Zahl von Kulturpflanzen zur Beseitigung unerwünschter Wildgräser oder grasartiger Kulturpflanzen, sofern sie an gewissen Standorten unerwünscht sind, eingesetzt werden. In Betracht kommen beispielsweise folgende Kulturen :

| Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var, silvestris | Rübsen |
| Camellia sinensis | Teestrauch |
| Carthamus tinctorius | Saflor - Färberdistel |
| Caryg illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |

| Name | Deutscher Name |
|---|---|
| Elaeis guineensis | Ölpalme |
| Fragaria vesca | Erdbeere |
| Glyoine max | Sojabohne |
| Gossypium hirsutum<br>(Gossypium arboreum<br>Gossypium herbaceum<br>Gossypium vitifolium) | Baumwolle |
| Helianthus annuus | Sonnenblume |
| Helianthus tuberosus | Topinambur |
| Hevea brasiliensis | Parakautschukbaum |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum<br>(N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Petroselinum crispum<br>spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sasamum indicum | Sesam |
| Solanum tuberosum | Kartoffel |
| Sorghum bicolor (s. vulgare) | Mohrenhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Cyclohexenonderivate der Formel I mit zahlreichen Vertretern anderer herbizider oder wachstumsregulierender Wirkstoffgruppen gemischt und gemeinsam ausgebracht werden. Beispielsweise kommen als

Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexenonderivate anderer Struktur und andere herbizide Wirkstoffe in Betracht.

Außerdem kann es von Nutzen sein, die Cyclohexenone der Formel I bzw. sie enthaltende herbizide Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nicht-phytotoxische Öle und Ölkonzentrate zugesetzt werden.

**Patentansprüche**

1. Cyclohexenonderivate der Formel

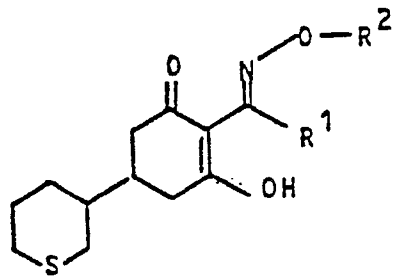

(I)

in der

$R^1$ $C_1$-$C_4$-Alkyl und

$R^2$ $C_3$-$C_5$-Chloralkenyl

bedeuten, sowie Salze dieser Verbindungen.

2. Cyclohexenonderivate der Formel I gemäß Anspruch 1, dadurch gekennzeichnet, daß $R^2$ einen 3-Chlor-prop-2-enylrest bedeutet.

3. Cyclohexenonderivat der Formel I gemäß Anspruch 1, dadurch gekennzeichnet, daß $R^1$ n-Propyl und $R^2$ trans-3-Chlor-prop-2-enyl bedeuten.

4. Verfahren zur Herstellung eines Cyclohexenonderivates der Formel I gemäß Anspruch 1, dadurch gekennzeichnet, daß man eine Verbindung der Formel

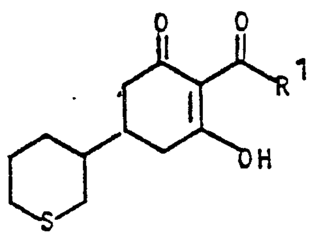

(II)

in der $R^1$ die in Anspruch 1 genannte Bedeutung hat,

a) mit einer Ammoniumverbindung der Formel $R^2ONH_3Y$, in der $R^2$ die in Anspruch 1 genannte Bedeutung hat und Y ein beliebiges Anion bedeutet, in einem inerten Lösungsmittel bei einer Temperatur zwischen 0 und 80 °C gegebenenfalls in Gegenwart einer Hilfsbase umsetzt oder

b) mit einem gegebenenfalls in wäßriger Lösung vorliegenden Hydroxylamin der Formel $R^2ONH_2$, wobei $R^2$ die im Anspruch 1 genannte Bedeutung hat, bei Temperaturen zwischen 0 und 70 °C gegebenenfalls in Gegenwart eines Lösungsmittels umsetzt, oder

c) mit unsubstituiertem Hydroxylammoniumsalz $NH_2OH \cdot HY$, wobei Y obige Bedeutung hat, bei 0 bis 80 °C in Gegenwart eines Lösungsmittels und einer Hilfsbase umsetzt und das so erhaltene Oxim mit einem Alkylierungsmittel $R^2$-Y', wobei Y' eine Abgangsgruppe bedeutet und $R^2$ die im Anspruch 1 genannte Bedeutung hat, alkyliert.

5. Herbizid, enthaltend ein Cyclohexenonderivat der Formel I gemäß Anspruch 1.

6. Herbizid, enthaltend inerte Zusatzstoffe und ein Cyclohexenonderivat der Formel

(Siehe Formel Seite 11 f.)

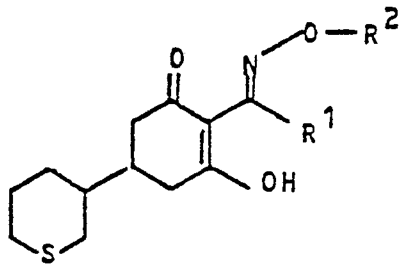

(I)

in der

$R^1$ $C_1$-$C_4$-Alkyl und

$R^2$ $C_3$-$C_5$-Chloralkenyl bedeuten,

oder Salze dieser Verbindungen.

7. Herbizid nach Anspruch 6, dadurch gekennzeichnet, daß es ein Cyclohexenonderivat der Formel I enthält, wobei $R^2$ einen 3-Chlor-prop-2-enylrest bedeutet.

8. Herbizid nach Anspruch 1, dadurch gekennzeichnet, daß es ein Cyclohexenonderivat der Formel I enthält, in der $R^1$ n-Propyl und $R^2$ 3-Chlor-prop-2-enyl bedeutet.

9. Verfahren zur Bekämpfung unerwünschten Pflanzenwuchses, dadurch gekennzeichnet, daß man die unerwünschten Pflanzen und/oder die von unerwünschten Pflanzenwachstum freizuhaltende Fläche mit einer herbizid wirksamen Menge eines Cyclohexenonderivates der Formel I gemäß Anspruch 1 behandelt.

**Claims**

1. A cyclohexenone derivative of the formula

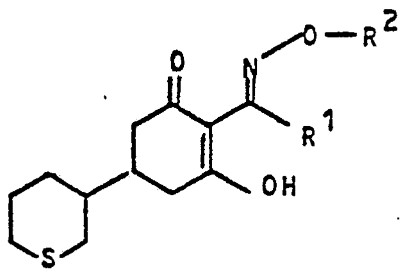

(I)

where

$R^1$ is $C_1$-$C_4$-alkyl and

$R^2$ is $C_3$-$C_5$-chloroalkenyl,

and salts thereof.

2. A cyclohexenone derivative of the formula I as claimed in claim 1, wherein $R^2$ is 3-chloroprop-2-enyl.

3. A cyclohexenone derivative of the formula I as claimed in claim 1, wherein $R^1$ is n-propyl and $R^2$ is trans-3-chloroprop-2-enyl.

4. A process for the preparation of a cyclohexenone derivative of the formula I as claimed in claim 1, wherein a compound of the formula

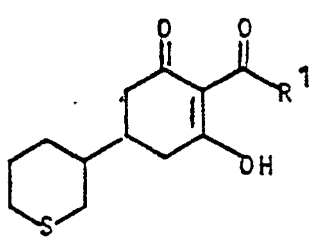

(II)

where $R^1$ has the meanings given in claim 1, is reacted with

a) an ammonium compound of the formula $R^2ONH_3Y$ where $R^2$ has the meanings given in claim 1 and Y is any anion, in an inert solvent, at from 0 to 80 °C and in the presence or absence of an auxiliary base, or

b) a hydroxylamine — if desired in aqueous solution — of the formula $R^2ONH_2$ where $R^2$ has the meanings given in claim 1, at from 0 to 70 °C and in the presence or absence of a solvent, or

c) an unsubstituted hydroxylammonium salt $NH_2OH \cdot HY$ where Y has the above meaning, at from 0 to 80 °C in the presence of a solvent and an auxiliary base, and the oxime thus obtained is alkylated with an alkylating agent $R^2$-Y' where Y' is a leaving group and $R^2$ has the meanings given in claim 1.

5. A herbicide containing a cyclohexenone derivative of the formula I as claimed in claim 1.

6. A herbicide containing inert additives and a cyclohexenone derivative of the formula

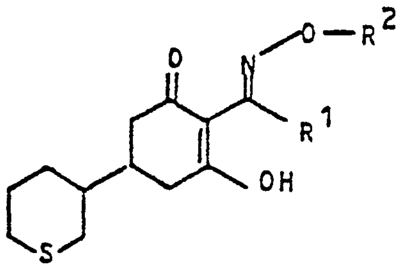

(I)

where

$R^1$ is $C_1$-$C_4$-alkyl and

$R^2$ is $C_3$-$C_5$-chloroalkenyl,

or a salt thereof.

7. A herbicide as claimed in claim 6, which contains a cyclohexenone derivative of the formula I where $R^2$ is 3-chloroprop-2-enyl.

8. A herbicide as claimed in claim 1, which contains a cyclohexenone derivative of the formula I where $R^1$ is n-propyl and $R^2$ is 3-chloroprop-2-enyl.

9. A process for controlling unwanted plant growth, wherein the unwanted plants and/or the area to be kept free from unwanted plant growth are treated with a herbicidally effective amount of a cyclohexenone derivative of the formula I as claimed in claim 1.

## Revendications

1. Dérivés de cyclohexenone de formule

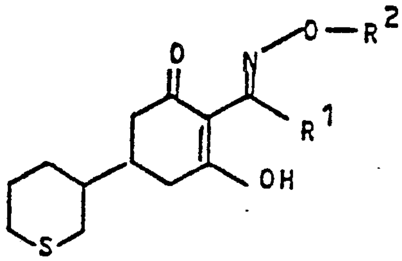

(I)

dans laquelle

$R^1$ représente alkyle en $C_1$-$C_4$ et

$R^2$ chloralcényle en $C_3$-$C_5$

ainsi que des sels de ces composés.

2. Dérivés de cyclohexenone de formule I, selon la revendication 1, caractérisés par le fait que $R^2$ représente un reste 3-chloro-prop-2-enyle.

3. Dérivés de cyclohexenone de formule I, selon la revendication 1, caractérisés par le fait que $R^1$ représente n-propyle et $R^2$ trans-3-chloro-prop-2-enyle.

4. Procédé de préparation d'un dérivé de cyclohexenone de formule I, selon la revendication 1, caractérisé par le fait que l'on fait réagir un composé de formule

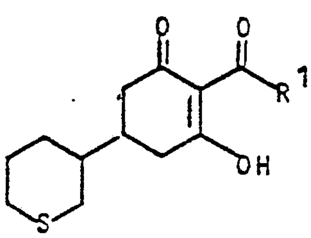

(II)

dans laquelle $R^1$ a la signification donnée dans la revendication 1

a) avec un dérivé d'ammonium de formule $R^2ONH_3Y$, dans laquelle $R^2$ a la signification donnée dans la revendication 1 et Y représente un anion quelconque, dans un solvant inerte, à une température comprise entre 0 et 80 °C, éventuellement en présence d'une base auxiliaire ou

b) avec une hydroxylamine de formule $R^2ONH^2$, se trouvant éventuellement en solution aqueuse, $R^2$ ayant la signification donnée dans la revendication 1, à des températures comprises entre 0 et 70 °C, éventuellement en présence d'un solvant, ou

c) avec un sel d'hydroxylamine non substitué $NH_2OH \cdot HY$, Y ayant la signification ci-dessus, à une température de 0 à 80 °C, en présence d'un solvant et d'une base auxiliaire, et on alkyle l'oxime ainsi obtenu avec un agent d'alkylation $R^2$-Y', Y' représentant un groupe éliminable et $R^2$ ayant la signification indiquée dans la revendication 1.

5. Herbicide contenant un dérivé de cyclohexenone de formule I, selon la revendication 1.

6. Herbicide, contenant des additifs inertes et un dérivé de cyclohexenone de formule

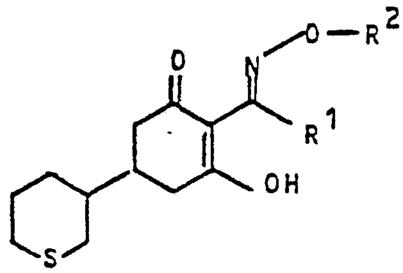

(I)

dans laquelle

$R^1$ représente alkyle en $C_1$-$C_4$ et

$R^2$ chloralcényle en $C_3$-$C_5$

ainsi que des sels de ces composés.

7. Herbicide selon la revendication 6, caractérisé par le fait qu'il contient un dérivé de cyclohexenone de formule I, $R^2$ représentant un reste 3-chloro-prop-2-enyle.

8. Herbicide selon la revendication 1, caractérisé par le fait qu'il contient un dérivé de cyclohexenone de formule I, dans laquelle $R^1$ représente n-propyle et $R^2$, 3-chloroprop-2-enyle.

9. Procédé de lutte contre la croissance indésirable des plantes, caractérisé par le fait que l'on traite les plantes indésirables et/ou les surfaces à maintenir exemptes d'une croissance de plantes indésirables, avec une quantité efficace au point de vue herbicide d'un dérivé de cyclohexenone de formule I selon la revendication 1.